# EUROPEAN PATENT SPECIFICATION

(11) **EP 3 666 963 B1**
(45) Date of publication and mention of the grant of the patent: **01.12.2021**
(21) Application number: 19202940.3
(22) Date of filing: 18.12.2015
(51) Int. Cl.: D06F 58/20, D06F 58/24, D06F 58/10

(54) **CLOTHES TREATMENT APPARATUS**
GEWEBEBEHANDLUNGSVORRICHTUNG
APPAREIL DE TRAITEMENT DE VÊTEMENTS

(30) Priority: 19.12.2014 KR 20140184452
(43) Date of publication of application: 17.06.2020
(62) Divisional of application: 15201376.9
(73) Proprietor: LG Electronics Inc., Seoul 07336 (KR)
(72) Inventor: PARK, Sunghoo, Seoul 08592 (KR); DOH, Youngjin, Seoul 08592 (KR); PARK, Hyoungsup, Seoul 08592 (KR)
(74) Representative: Vossius & Partner Patentanwälte Rechtsanwälte mbB

(56) References cited:
- EP-A1- 1 659 207
- EP-A1- 2 508 668
- WO-A1-2007/126225
- WO-A2-2009/020313
- WO-A2-2009/123407
- DE-U1- 8 805 385
- GB-A- 2 511 432
- KR-A- 20140 016 093

## Description

The present invention relates to a clothes treatment apparatus.

Clothes treatment apparatuses are apparatuses that treat clothes, e.g. wash and dry clothes and smooth wrinkles in clothes, at home or at laundromats.

Clothes treatment apparatuses may be classified into a washer for washing clothes, a dryer for drying clothes, a washer/dryer having both a washing function and a drying function, a refresher for refreshing clothes, and a steamer for removing unnecessary wrinkles in clothes.

The refresher is an apparatus that keeps clothes comfortable and fresh. The refresher functions to dry clothes, to supply fragrance to clothes, to prevent the occurrence of static electricity in clothes, or to remove wrinkles from clothes.

The steamer is an apparatus that simply supplies steam to clothes in order to remove wrinkles from the clothes. Unlike a general iron, the steamer removes wrinkles from the clothes without directly applying heat to the clothes.

A clothes treatment apparatus having both functions of a refresher and a steamer may remove wrinkles from clothes received in the clothes treatment apparatus, and may additionally deodorize the clothes, using steam and hot air.

An example of such a conventional treatment apparatus is disclosed in Korean Patent Application Publication No. 10-2014-0016093. Another example is disclosed in WO 2009/123407.

Therefore, the present invention has been made in view of the above problems, and it is an object of the present invention to provide a clothes treatment apparatus that is capable of directly sensing the level of water stored in a tank.

It is another object of the present invention to provide a clothes treatment apparatus that is capable of enabling a user to immediately check the deficiency of water during the operation of the clothes treatment apparatus.

The objects are solved by the independent claim. The dependent claims relate to further aspects of the invention.

In accordance with an aspect of the present invention, the above and other objects can be accomplished by the provision of a clothes treatment apparatus including a cabinet partitioned into a treatment chamber for allowing clothes to be hung therein, a cycle chamber for allowing machinery to be installed therein, and a tank installation space for allowing a removable tank to be installed therein, a door for opening and closing the cabinet, a steam unit disposed in the cycle chamber for supplying steam into the treatment chamber, a heat pump unit disposed in the cycle chamber for circulating air in the treatment chamber and conditioning the air that is circulated, a water supply tank disposed in the tank installation space, such that the water supply tank is separably installed in the tank installation space and is connected to the steam unit, for supplying water to the steam unit, a drainage tank disposed in the tank installation space, such that the drainage tank is separably installed in the tank installation space, for storing condensed water generated in at least one selected from between the treatment chamber and the heat pump unit, a water supply level sensor disposed in the water supply tank configured to directly sense the level of water stored in the water supply tank, and a drainage level sensor disposed in the drainage tank configured to directly sense the level of water stored in the drainage tank.

The clothes treatment apparatus may further include a partition plate for partitioning the interior of the cabinet into upper and lower interior parts such that the treatment chamber and the cycle chamber are partitioned from each other, and a tank module frame for partitioning the interior of the cabinet below the partition plate into front and rear interior parts such that the cycle chamber and the tank installation space are partitioned from each other.

The tank installation space may be formed so as to face the door.

The water supply tank and the drainage tank may be arranged parallel to each other in rightward and leftward directions.

The clothes treatment apparatus may further include a tank support bar disposed between the tank installation space and the door, wherein at least one selected from between the water supply tank and the drainage tank may be disposed so as to be placed on the tank support bar.

The at least one selected from between the water supply tank and the drainage tank may be provided with a tank support end, the tank support end interfering with the tank support bar, the tank support end being concavely recessed.

The water supply tank or the drainage tank, placed on the tank support bar, may form a continuous surface with the tank support bar.

The upper side of at least one selected from between the water supply tank and the drainage tank may be round such that, when the at least one selected from between the water supply tank and the drainage tank is separated, interference between the at least one selected from between the water supply tank and the drainage tank and the partition plate is minimized.

At least one selected from between the water supply tank and the drainage tank may be provided with a grip, the grip being formed at the at least one selected from between the water supply tank and the drainage tank such that the grip is concave from the front to the rear thereof.

The water supply level sensor may include a float case fixed in the water supply tank, a float installed in the float case such that the float is movable upward and downward in the float case by buoyancy, and a sensor installed at the cabinet configured to sense a magnetic force of the float.

The sensor may be installed in any one selected from between the cycle chamber and the tank installation space.

The float case of the water supply level sensor may be disposed at a position at which water remains in an amount that is sufficient to be supplied to the steam unit during one cycle.

The drainage level sensor may include a float case fixed in the drainage tank, a float installed in the float case such that the float is movable upward and downward in the float case by buoyancy, and a sensor installed at the cabinet configured to sense a magnetic force of the float.

The sensor may be installed in any one selected from between the cycle chamber and the tank installation space.

The float case of the drainage level sensor may be disposed at a position at which capacity remains to store an amount of condensed water generated during the cycle in the at least one selected from between the treatment chamber and the heat pump unit.

The water supply tank or the drainage tank may include a tank body that is open at the front thereof, the upper side of the surface of the tank body that is inserted into the tank installation space being round, a tank cover coupled to the front of the tank body, the tank cover being provided with a grip, the grip being concavely formed inward, and a check valve installed in the tank body for opening and closing a flow channel extending from the tank body to the outside.

The clothes treatment apparatus may further include a tank support bar disposed between the tank installation space and the door, wherein at least one selected from between the water supply tank and the drainage tank may be disposed so as to be placed on the tank support bar, and the at least one selected from between the water supply tank and the drainage tank may be provided with a tank support end, the tank support end being placed on the upper side of the tank support bar such that the tank support end interferes with the tank support bar, the tank support end being recessed rearward.

The check valve installed in the water supply tank may be disposed at the lower side of the tank body, and may be connected to the steam unit to supply water to the steam unit.

The clothes treatment apparatus may further include a water hole formed at the upper side of the tank body and a water hole cover for opening and closing the water hole.

The water supply tank or the drainage tank may further include a float installation part formed in the tank body, the tank body and the tank cover may be manufactured by insert injection molding using die slide injection (DSI), and the water supply level sensor or the drainage level sensor may be installed in the float installation part by insert injection molding using DSI.

### BRIEF DESCRIPTION OF THE DRAWINGS

The embodiments will be described in detail with reference to the following drawings in which like reference numerals refer to like elements wherein:
FIG. 1 is a perspective view of a clothes treatment apparatus according to a first embodiment of the present invention;
FIG. 2 is an exploded perspective view of a cycle assembly according to a first embodiment of the present invention;
FIG. 3 is a perspective view of the cycle assembly according to the first embodiment of the present invention;
FIG. 4 is an exploded perspective view of a water supply tank shown in FIG. 1;
FIG. 5 is a partially exploded perspective view of the water supply tank shown in FIG. 1;
FIG. 6 is a sectional perspective view of a check assembly shown in FIG. 5;
FIG. 7 is a side sectional view of the water supply tank shown in FIG. 1;
FIG. 8 is a perspective view of a drainage tank shown in FIG. 1;
FIG. 9 is a partially exploded perspective view of the drainage tank shown in FIG. 1;
FIG. 10 is a side sectional view of the drainage tank shown in FIG. 1; and
FIG. 11 is a block diagram of the clothes treatment apparatus shown in FIG. 1.

### DETAILED DESCRIPTION OF THE PREFERRED EMBODIMENTS

The present invention will be described in detail with reference to the accompanying drawings.

In the following description of the present invention, a detailed description of known functions or configurations incorporated herein will be omitted when it may make the subject matter of the present invention rather unclear. The same terms may be denoted by different reference numerals if the terms indicate different parts.

The terms used in the following description are terms defined taking into consideration the functions obtained in accordance with the present invention. The definitions of these terms should be determined based on the whole content of this specification because they may be changed in accordance with the intentions of users, such as experimenters and measurers, or usual practices.

In this specification, the terms "first," "second," etc. are used to describe various elements. However, the elements are not limited by the terms. The terms are used only to distinguish one element from another element. For example, a first element may be named a second element, and a second element may be named a first element, without departing from the scope of right of the present invention. It will be understood that the term "and/or" refers to one or more possible combinations of specified relevant items and includes such combinations.

The terms used in this specification are provided only to explain specific embodiments, but are not intended to restrict the present invention. A singular representation may include a plural representation unless it represents a definitely different meaning from the context.

Unless otherwise defined, all terms, including technical and scientific terms, used in this specification have the same meaning as commonly understood by a person having ordinary skill in the art to which the present invention pertains. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

In addition, the terms "comprises" and "includes" described herein should be interpreted not to exclude other elements but to further include such other elements since the corresponding elements may be inherent unless mentioned otherwise.

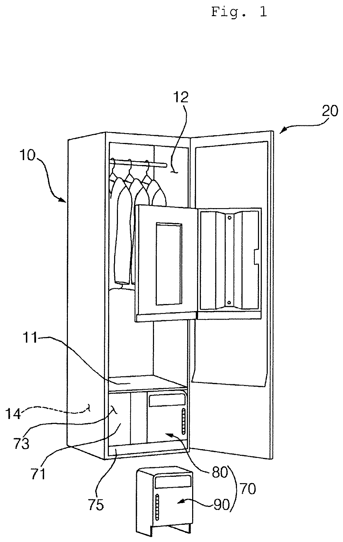

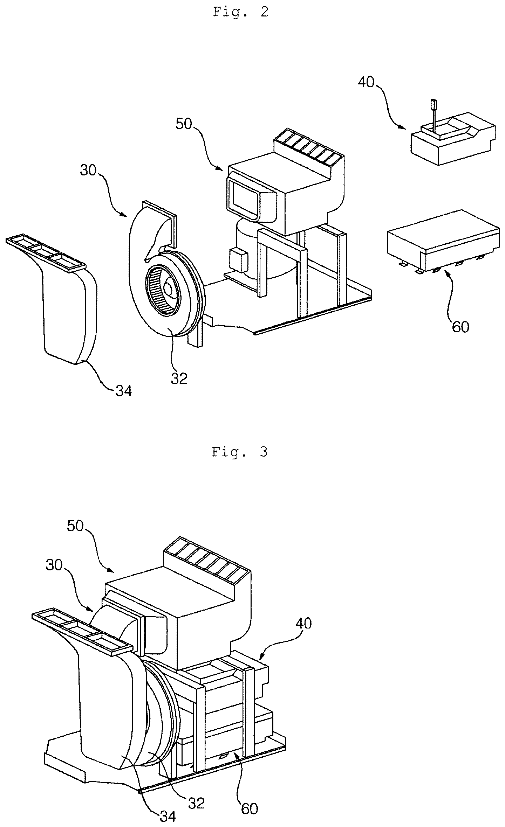

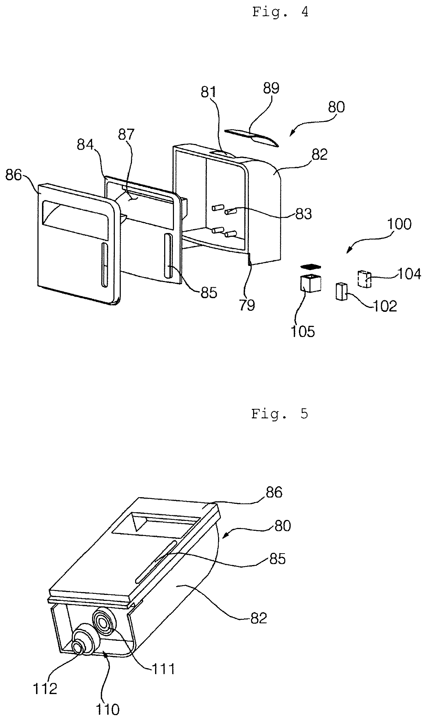

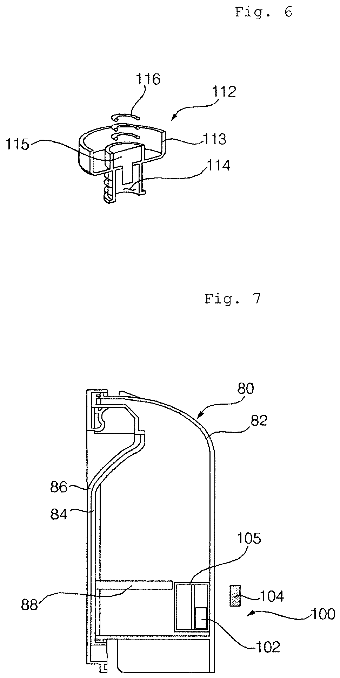

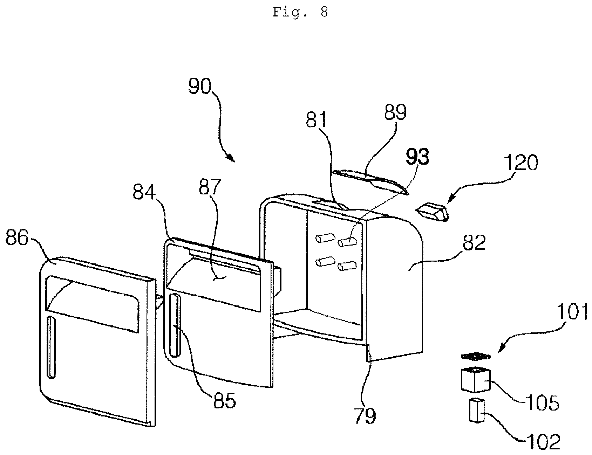

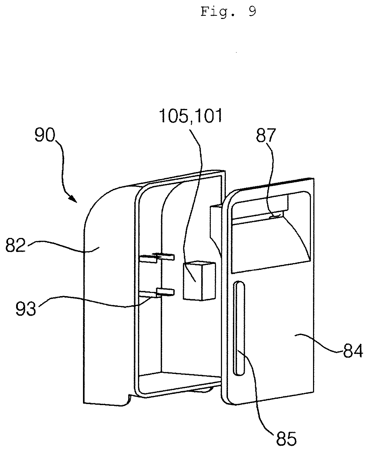

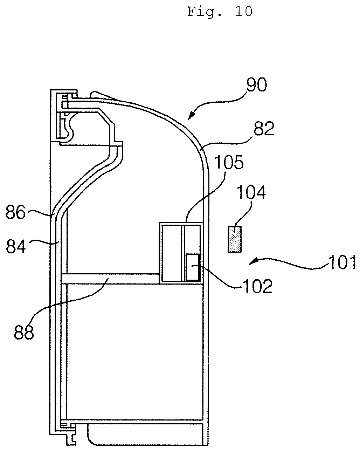

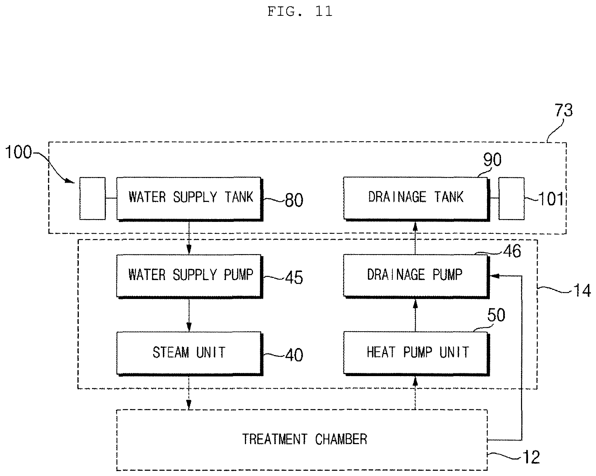

FIG. 1 is a perspective view of a clothes treatment apparatus according to a first embodiment of the present invention, FIG. 2 is an exploded perspective view of a cycle assembly according to a first embodiment of the present invention, FIG. 3 is a perspective view of the cycle assembly according to the first embodiment of the present invention, FIG. 4 is an exploded perspective view of a water supply tank shown in FIG. 1, FIG. 5 is a partially exploded perspective view of the water supply tank shown in FIG. 1, FIG. 6 is a sectional perspective view of a check assembly shown in FIG. 5, FIG. 7 is a side sectional view of the water supply tank shown in FIG. 1, FIG. 8 is a perspective view of a drainage tank shown in FIG. 1, FIG. 9 is a partially exploded perspective view of the drainage tank shown in FIG. 1, FIG. 10 is a side sectional view of the drainage tank shown in FIG. 1, and FIG. 11 is a block diagram of the clothes treatment apparatus shown in FIG. 1.

The clothes treatment apparatus according to this embodiment includes a cabinet 10 and a door 20 configured to open and close the front of the cabinet 10.

The interior of the cabinet 10 is partitioned into upper and lower interior parts by a partition plate 11. A treatment chamber 12, in which clothes are hung, is defined in the interior of the cabinet 10 above the partition plate 11. A cycle chamber 14, in which machinery is installed, is defined in the interior of the cabinet 10 below the partition plate 11.

Clothes are hung in the treatment chamber 12. In the treatment chamber 12, wrinkles in the clothes are smoothed, or the clothes are deodorized, by the circulation of steam or air.

A blowing unit 30 for circulating air in the treatment chamber 12, a steam unit 40 for supplying steam into the treatment chamber 12, a heat pump unit 50 for conditioning air in the treatment chamber 12, and a control unit 60 for controlling the respective units 30, 40, and 50 are installed in the cycle chamber 14.

In this embodiment, an assembly of machinery, including the blowing unit 30, the steam unit 40, the heat pump unit 50, and the control unit 60, which are required to perform respective cycles of the clothes treatment apparatus, is defined as a cycle assembly.

The blowing unit 30 includes a blowing fan 32 and an inlet duct 34.

The inlet duct 34 is installed at the suction side of the blowing fan 32 to guide air in the treatment chamber 12 to the blowing fan 32.

The blowing fan 32 is rotated to blow air. The blowing fan 32 suctions air from the treatment chamber 12, and discharges the suctioned air to the heat pump unit 50.

When the steam unit 40 is powered on, heat is generated from the steam unit 40. The steam unit 40 converts water supplied from a water supply tank 80, which will be described hereinafter, into steam. The generated steam is discharged into the treatment chamber 12.

In this embodiment, a flow channel is defined such that the steam flows into the treatment chamber 12 via the heat pump unit 50.

The heat pump unit 50 constitutes a heat pump cycle including a compressor, a condenser, an evaporator, and an expansion valve. Based on the operation mode of the heat pump unit 50, cooled air or heated air may be discharged into the treatment chamber 12.

In particular, the heat pump unit 50 may dehumidify air supplied from the blowing unit 30.

A tank module 70 for storing water is installed in front of the cycle chamber 14. The tank module 70 includes a water supply tank 80 for supplying water to the steam unit 40 and a drainage tank 90 for gathering and storing condensed water that is generated in the treatment chamber 12.

Water from the water supply tank 80 flows to the steam unit 40 via a water supply pump 45.

Water that is condensed in the treatment chamber 12, flows to the lower side of the treatment chamber 12 due to gravity, and is then pumped to the drainage tank 90 by a drainage pump 46. Water that is condensed in the heat pump unit 50 also flows to the drainage tank 90 via the drainage pump 46.

The water supply pump 45 or the drainage pump 46 is controlled by the control unit 60.

In this embodiment, a tank module frame 71 is installed in front of the inlet duct 34.

A tank installation space 73 is defined between the tank module frame 71 and the door 20. The tank module frame 71 is coupled to the partition plate 11 to isolate the cycle chamber 14 from the outside.

A tank support bar 75, which interferes with at least one selected from between the water supply tank 80 and the drainage tank 90, is installed in front of the tank installation space 73.

The tank support bar 75 prevents the water supply tank 80 or the drainage tank 90 from being unintentionally separated from the tank installation space 73. The tank support bar 75 supports the front of the water supply tank 80 and the front of the drainage tank 90.

When the door 20 is opened and closed, therefore, the water supply tank 80 and the drainage tank 90 are prevented from being separated from the tank installation space 73.

In this embodiment, the lower end of the water supply tank 80 is placed on the upper end of the tank support bar 75, and the lower end of the drainage tank 90 is placed on the upper end of the tank support bar 75.

A tank support end 79, which interferes with the tank support bar 75, is formed on at least one selected from between the water supply tank 80 and the drainage tank 90.

The tank support end 79 is concavely recessed.

The front of the tank support bar 75 and the front of the water supply tank 80 may form a continuous surface due to the tank support end 79. In addition, the front of the tank support bar 75 and the front of the drainage tank 90 may form a continuous surface due to the tank support end 79

The water supply tank 80 and the drainage tank 90 are disposed in the tank installation space 73 such that the water supply tank 80 and the drainage tank 90 are arranged parallel to each other in rightward and leftward directions.

When the door 20 is opened, the water supply tank 80 and the drainage tank 90 are exposed to a user.

The water supply tank 80 and the drainage tank 90 may be withdrawn by the user.

The water supply tank 80 and the drainage tank 90 may be separated from the tank module frame 71. The water supply tank 80 and the drainage tank 90 may be separably mounted in the tank installation space 73.

The water supply tank 80 is connected to the steam unit 40 to supply water to the steam unit 40. The drainage tank 90 is connected to the treatment chamber 12 to store water discharged from the treatment chamber 12 or the heat pump unit 50.

The water supply tank 80 includes a tank body 82, which is open at the front thereof, a tank cover 84 coupled to the front of the tank body 82, a decorative cover 86 coupled to the tank cover 84, a water supply check valve 110 installed in the tank body 82 for opening and closing a flow channel connected with the steam unit 40, and a water supply level sensor 100 for sensing the level of water stored in the tank body 82.

The front of the tank body 82 is open. The water supply level sensor 100 is disposed in the tank body 82.

The upper end of the tank body 82 is round at the rear side thereof.

When the tank body 82 is separated, interference between the tank body 82 and the partition plate 11 is minimized.

The user may easily pull and withdraw the water tank 80, which is disposed at the lower side of the clothes treatment apparatus, due to the round shape of the tank body 82.

In this embodiment, the water supply level sensor 100 includes a float 102 installed in the tank body 82 such that the float 102 can move upward and downward based on the level of water stored in the tank body 82, a float cabinet 105 installed in the tank body 82 in a state in which the float 102 is disposed in the float cabinet 105, and a sensor 104 installed at the tank module frame 71 to sense the float 102.

The float 102 has a magnet. The sensor 104 senses the magnetic force of the magnet.

The sensor 104 may be installed at the front or rear of the tank module frame 71.

The sensor 104 may be installed through the tank module frame 71.

Consequently, the sensor 104 may be located in any one selected from among the cycle chamber 14, the tank installation space 73, and the tank module frame 71.

The float 102, which is installed in the water supply tank 80, is flush with the sensor 104. When the level of water stored in the water supply tank 80 is lowered, the float 102 moves lower than the sensor 104. When the sensor 104 fails to sense the float 102, therefore, the control unit 60 outputs a water deficiency signal. Even when the water deficiency signal is output, it is possible to supply a sufficient amount of steam during a cycle that is currently being performed.

Since the sensor 104 constantly senses the float 102, the control unit 60 may determine whether the water supply tank 80 is mounted.

For example, when the water supply tank 80 is not mounted, or when water is deficient, the control unit 60 outputs a water deficiency signal.

When the user manipulates the clothes treatment apparatus in a state in which the water deficiency signal is output, therefore, the control unit 60 performs control such that the clothes treatment apparatus is not operated and outputs a water deficiency signal. At this time, the user may check the water supply tank 80.

A float installation part 83, at which the float 102 is installed, is formed at the inside of the tank body 82. The float cabinet 105 is installed at the float installation part 83. The float 102 may move upward and downward along the float cabinet 105 by buoyancy.

In this embodiment, the float 102 is installed at the minimum level of water stored in the water supply tank 80, at which it is possible to supply an amount of steam corresponding to one cycle. Even when the sensor 104 fails to sense the float 102, and therefore the control unit 60 outputs a water deficiency signal, it is possible to supply an amount of steam corresponding to at least one cycle.

That is, even when a water deficiency signal is sensed during the supply of steam, it is possible to supply a sufficient amount of steam until a cycle that is currently being performed is completed.

The float cabinet 105, in which the float 102 is mounted, is manufactured by insert injection molding at the time of die slide injection (DSI) of the tank cover 84 and the tank body 82.

Die slide injection (DSI) is a molding technology that has been developed for blow molding or molding of thin products. DSI conveys various advantages in that no post-processing, such as adhesion or assembly, is necessary after injection molding, it is possible to adjust the thickness of a wall more easily than when blow molding or gas molding, it is possible to provide an excellent surface shape or high dimensional accuracy, and it is possible to perform DSI more easily than double injection or blow molding. The manufacturing of products using DSI is ordinarily known in the art to which the present invention pertains, and therefore a detailed description thereof will be omitted.

The tank body 82 and the tank cover 84 are manufactured by insert injection molding using DSI. During the manufacturing of the tank body 82 and the tank cover 84, the float cabinet 105 is installed in the tank body 82 and the tank cover 84 by insert injection molding. During the manufacturing of the tank body 82 and the tank cover 84, the edge of the tank cover 84 is integrally coupled to the edge of the tank body 82.

The tank cover 84 has a window 85, through which the user may check the level of water in the tank body 82. In addition, a grip 87, into which the user may insert his/her hand in order to hold the tank cover 84, is concavely formed at the tank cover 84.

The grip 87 is formed at the tank cover 84 such that the grip 87 is concave from the front to the rear thereof.

A sensor fixing part 88 is formed at the inside of the tank cover 84. The sensor fixing part 88 protrudes from the inside of the tank cover 84. When the tank cover 84 and the tank body 82 are coupled to each other, the sensor fixing part 88 comes into tight contact with the float cabinet 105.

Since the sensor fixing part 88 tightly contacts the float cabinet 105, the float cabinet 105 is prevented from being separated from the float installation part 83.

The sensor fixing part 88 may be integrally formed with the tank cover 84.

The decorative cover 86 is formed to have a shape that is capable of covering the front of the tank cover 84. In addition, the decorative cover 86 is formed to have a shape corresponding to the shape of the tank cover 84.

A water hole 81 is formed at the upper side of the tank body 82. In addition, a water hole cover 89 for opening and closing the water hole 81 is disposed at the upper side of the tank body 82.

The water hole cover 89 is made of a flexible material exhibiting high elasticity. One end of the water hole cover 89 is fixed to the tank body 82, and the other end of the water hole cover 89 may be bent in order to open and close the water hole 81.

The water supply check valve 110 includes a check valve hole 111 formed at the lower side of the tank body 82 and a check assembly 112 coupled to the check valve hole 111 for regulating the water in the tank body 82.

The check assembly 112 includes a check housing 113 coupled into the check valve hole 111, the check housing 113 having a check flow channel 114, through which water flows into the check housing 113, a valve 115 disposed in the check housing 113 for opening and closing the check flow channel 114, and a check elastic member 116 disposed between the valve 115 and the tank body 82 for applying elastic force to the valve 115.

The small-diameter side of the valve 115 protrudes downward. When the valve 115 is placed on the tank module frame 71, the valve 115 may be pushed by the tank module frame 71, and may thus move upward. At this time, the check flow channel 114 is opened as the result of the movement of the valve 115. When the water supply tank 80 is separated from the tank module frame 71, the check flow channel 114 is closed by the elastic force of the check elastic member 116.

The drainage tank 90 is identical in function to the water supply tank 80. The drainage tank 90 is disposed alongside the water supply tank 80.

In the drainage tank 90, a drainage check valve 120 is installed at the rear side thereof, not at the lower side thereof, unlike the water supply tank 80.

The water supply tank 80 receives water through the water hole 81, and discharges water through the water supply check valve 110. The drainage tank 90 may receive condensed water through the drainage check valve 120, and may discharge condensed water through the water hole 81.

That is, the drainage check valve 120 of the drainage tank 90 may be disposed in a channel for receiving condensed water, not for discharging condensed water.

Unlike this embodiment, condensed water may fall into the drainage tank 90 through the water hole 81. In addition, condensed water may be automatically discharged through the drainage check valve 120.

Water that is condensed in the treatment chamber 12 and water that is condensed in the heat pump unit 50 are stored in the drainage tank 90.

A float installation part 93, at which the float cabinet 105 is installed, is formed in the drainage tank 90.

The float installation part 93 may be located at a height in the drainage tank 90 at which overflow does not occur even when an amount of condensed water that is generated during one cycle is stored therein.

That is, the float installation part 93 is located at a height in the drainage tank 90 at which overflow does not occur even when an amount of condensed water that is generated during one cycle is stored in the drainage tank 90.

When a drainage level sensor 101 of the drainage tank 90 senses a signal during the operation of the clothes treatment apparatus, therefore, the water in the drainage tank 90 does not overflow due to the condensed water that is additionally stored in the drainage tank 90.

The drainage level sensor 101 of the drainage tank 90 is located higher than the water supply level sensor 100 in the water supply tank 80.

The drainage level sensor 101 of the drainage tank 90 is identical in construction to the water supply level sensor 100 of the water supply tank 80. However, the drainage level sensor 101 of the drainage tank 90 is operated differently from the water supply level sensor 100 of the water supply tank 80.

For example, the sensor 104 of the drainage tank 90 does not sense the float 102 in a normal state. When the level of condensed water rises, the sensor 104 of the drainage tank 90 senses the float 102, which has been raised by buoyancy.

When the sensor 104 of the drainage tank 90 senses the float 102, the control unit 60 outputs a water drainage signal. When the water drainage signal is output, however, the overflow of condensed water does not occur during a cycle that is currently being performed.

As is apparent from the above description, the clothes treatment apparatus according to the present invention has the following effects.

It is possible to directly sense the amount of water stored in the water supply tank instead of estimating the amount of water stored in the water supply tank.

It is possible to sense the level of water stored in the water supply tank without delay.

It is possible to directly sense the level of water stored in the drainage tank without delay, thereby preventing water from overflowing the drainage tank.

In the clothes treatment apparatus according to the present invention, the water supply level sensor is installed at the level of water that is required to generate enough steam for at least one cycle. Consequently, it is possible to prevent the supply of water from being interrupted while steam is being generated.

In the clothes treatment apparatus according to the present invention, the drainage level sensor is installed at the level of water at which it is possible to store all of the water that is condensed during at least one cycle. Consequently, it is possible to prevent the condensed water from overflowing the drainage tank, or it is not necessary to drain the condensed water from the drainage tank, during the operation of the clothes treatment apparatus.

It will be apparent that, although the embodiments of the present invention have been described above with reference to the accompanying drawings, the present invention is not limited to the above-described specific embodiments, and therefore various modifications and variations can be made by those skilled in the art without departing from the gist of the appended claims. Thus, it is intended that the modifications and variations should not be understood independently of the technical prospect of the present invention. The above embodiments are therefore to be construed in all aspects as illustrative and not restrictive.

## Claims

1. A clothes treatment apparatus comprising:
a cabinet (10) partitioned into a treatment chamber (12) for allowing clothes to be hung therein, a cycle chamber (14) for allowing machinery to be installed therein, and a tank module frame (71) configured to allow a water supply tank (80) and a drainage tank (90) to be installed therein;
a door (20) for opening and closing the cabinet (10);
a steam unit (40) disposed in the cycle chamber (14) for supplying steam into the treatment chamber (12);
a heat pump unit (50) disposed in the cycle chamber (14) for circulating air in the treatment chamber (12) and conditioning the air that is circulated;
wherein the water supply tank (80) is separably installed in the tank module frame (71), and is connected to the steam unit (40), configured to supply water to the steam unit (40), and
wherein the drainage tank (90) is separably installed in the tank module frame (71), and is configured to store condensed water generated in at least on selected from between the treatment chamber (12) and the heat pump unit (50); **characterized in that** it further comprises
a water supply level sensor (100) configured to sense a level of water stored in the water supply tank (80);
a drainage level sensor (101) configured to sense a level of water stored in the drainage tank (90), and
the drainage level sensor (101) is located higher than the water supply level sensor (100).

2. The clothes treatment apparatus according to claim 1, wherein each of the water supply level sensor (100) and the drainage level sensor (101) comprises:
a float case (105) fixed in each of the water supply tank (80) and drainage tank (90);
a float (102) installed in the float case (105) such that the float (102) is movable upward and downward in the float case (105) by buoyancy; and
a sensor (104) installed on the tank module frame (71) and configured for sensing a magnetic force of the float (102).

3. The clothes treatment apparatus according to claim 2, wherein the sensor (104) of the water supply level sensor (100) is located at a corresponding height to the upper end of the float case (105) of the water supply level sensor (100), and the float (102) of the water supply level sensor (100) moves lower than the sensor (104) of the water supply level sensor (100) when the level of water stored in the water supply tank (80) is lowered.

4. The clothes treatment apparatus according to claims 2 or 3, wherein the float case (105) of the water supply tank (80) is installed at a lower inward surface of a rear side in the water supply tank (80).

5. The clothes treatment apparatus according to any one of claims 2 to 4, wherein the sensor (104) of the water supply level sensor (100) further determine whether the water supply tank (80) is mounted in the tank installation space (73).

6. The clothes treatment apparatus according to any one of claims 2 to 5, wherein the sensor (104) of the drainage level sensor (101) is located at a corresponding height to lower ends of the float case (105) of the drainage level sensor (101), and the float (102) of the drainage level sensor (101) moves higher than the sensor (104) of the drainage level sensor (101) when the level of water stored in the drainage tank (80) is increased.

7. The clothes treatment apparatus according to any one of claims 2 to 6, wherein the float case (105) of drainage level sensor (101) is installed at a rear side lower than the rounded upper end of drainage tank (90).

8. The clothes treatment apparatus according to any one of claims 2 to 7, wherein the sensor (104) of the drain level sensor (101) further determine whether the drainage tank (90) is mounted in the tank installation space (73).

9. The clothes treatment apparatus according to any one of claims 2 to 8, further comprising:
a control unit (60) located in the cycle chamber (14) and configured to control the steam unit (40) and the heat pump unit (50), based on each signal from each of the water supply level sensor (100) and the drainage level sensor (101).

10. The clothes treatment apparatus according to claim 9, wherein the clothes treatment apparatus fails to start and the control unit (60) is configured to output water deficiency signal when height of the float (102) of the water supply level sensor (100) is lower than height of the sensor (104) of the water supply level sensor (100) before the operation of the clothes treatment apparatus, and
wherein the control unit (60) fails to outputs water deficiency signal until finished, though height of the float (102) of the water supply level sensor (100) is lower than height of the sensor (104) of the water supply level sensor (100) during the operation of the clothes treatment apparatus.

11. The clothes treatment apparatus according to claim 9 or 10, wherein the control unit (60) is configured to determine the stored water in the water supply tank (80) is sufficient for operating the clothes treatment apparatus when the float (102) of the water supply level sensor (100) is located at the corresponding height of the sensor (104) of the water supply level sensor (100).

12. The clothes treatment apparatus according to any one of claims 9 to 11, wherein the clothes treatment apparatus fails to start and the control unit (60) is configured to output a water deficiency signal when height of the float (102) of the drainage level sensor (101) is higher than height of the sensor (104) of the drainage level sensor (101) before the operation of the clothes treatment apparatus, and wherein the control unit (60) fails to outputs a water deficiency signal until finished, though height of the float (102) of the drainage level sensor (101) is higher than height of the sensor (104) of the drainage level sensor (101) during the operation of the clothes treatment apparatus.

13. The clothes treatment apparatus according to any one of claims 9 to 12, wherein the control unit (60) is configured to determine whether the remained capacity of water in the drainage tank (90) is sufficient for operating the clothes treatment apparatus when the float (102) of the drainage level sensor (101) is located at the corresponding height of the sensor (104) of the drainage level sensor (101).

14. The clothes treatment apparatus according to any one of claims 1 to 13, wherein the tank module frame (71) is installed in front of an inlet duct (34) configured for guiding air from the treatment chamber (12) to the cycle chamber (14), and is configured to partition a tank installation space (73) defined in the tank module frame (71) and the cycle chamber (14), and wherein each of the water supply tank (80) and the drainage tank (90) is separably mounted in the tank installation space (73).

15. The clothes treatment apparatus according to claim 14, wherein each front side of the water supply tank (80) and the drainage tank (90) is exposed to a user when the door (20) is open, and each front side of the water supply tank (80) and the drainage tank (90) faces the inner side of door (20) when the door (20) is closed.

## Patentansprüche

1. Kleidungsbehandlungsvorrichtung, die aufweist:
einen Schrank (10), der in eine Behandlungskammer (12), um zu ermöglichen, dass Kleidung in ihr aufgehängt wird, eine Arbeitskammer (14), um zu ermöglichen, dass Anlagen darin installiert werden, und einen Behältermodulrahmen (71), der konfiguriert ist, um zu ermöglichen, dass ein Wasserversorgungsbehälter (80) und ein Wasserabführungsbehälter (90) darin installiert werden, unterteilt ist;
eine Tür (20) zum Öffnen und Schließen des Schranks (10);
eine Dampfeinheit (40), die in der Arbeitskammer (14) angeordnet ist, um Dampf in die Behandlungskammer (12) zuzuführen;
eine Wärmepumpeneinheit (50), die in der Arbeitskammer (14) angeordnet ist, um Luft in der Behandlungskammer (12) zu zirkulieren und die Luft, die zirkuliert wird, zu klimatisieren;
wobei der Wasserversorgungsbehälter (80) trennbar in dem Behältermodulrahmen (71) installiert ist und mit der Dampfeinheit (40) verbunden ist, die konfiguriert ist, um Wasser an die Dampfeinheit (40) zuzuführen, und
wobei der Wasserabführungsbehälter (90) trennbar in dem Behältermodulrahmen (71) installiert ist und konfiguriert ist, um Kondenswasser zu lagern, das in wenigstens einer ausgewählten der Behandlungskammer (12) und der Wärmepumpeneinheit (50) erzeugt wird;
**dadurch gekennzeichnet, dass** sie ferner aufweist:
einen Wasserversorgungsstandsensor (100), der konfiguriert ist, um einen Stand des in dem Wasserversorgungsbehälter (80) gelagerten Wassers abzutasten;
einen Wasserabführungsstandsensor (101), der konfiguriert ist, um einen Stand des in dem Wasserabführungsbehälter (90) gelagerten Wassers abzutasten; und
der Wasserabführungsstandsensor (101) höher angeordnet ist als der Wasserversorgungsstandsensor (100).

2. Kleidungsbehandlungsvorrichtung nach Anspruch 1, wobei der Wasserversorgungsstandsensor (100) und der Wasserabführungsstandsensor (101) jeweils aufweisen:
ein Schwimmergehäuse (105), das in jedem des Wasserversorgungsbehälters (80) und des Wasserabführungsbehälters (90) befestigt ist;
einen Schwimmer (102), der in dem Schwimmergehäuse (105) installiert ist, so dass der Schwimmer (102) durch Auftrieb in dem Schwimmergehäuse (105) aufwärts und abwärts beweglich ist; und
einen Sensor (104), der auf dem Behältermodulrahmen (71) installiert ist und konfiguriert ist, um eine Magnetkraft des Schwimmers (102) abzutasten.

3. Kleidungsbehandlungsvorrichtung nach Anspruch 2, wobei der Sensor (104) des Wasserversorgungsstandsensors (100) auf einer Höhe angeordnet ist, die dem oberen Ende des Schwimmergehäuses (105) des Wasserversorgungsstandsensors (100) entspricht, und der Schwimmer (102) des Wasserversorgungsstandsensors (100) sich tiefer bewegt als der Sensor (104) des Wasserversorgungsstandsensors (100), wenn der Stand des in dem Wasserversorgungsbehälter (80) gelagerten Wassers gesenkt wird.

4. Kleidungsbehandlungsvorrichtung nach Anspruch 2 oder 3, wobei das Schwimmergehäuse (105) des Wasserversorgungsbehälters (80) an einer unteren einwärtigen Oberfläche einer Rückseite in dem Wasserversorgungsbehälter (80) installiert ist.

5. Kleidungsbehandlungsvorrichtung nach einem der Ansprüche 2 bis 4, wobei der Sensor (104) des Wasserversorgungsstandsensors (100) ferner bestimmt, ob der Wasserversorgungsbehälter (80) in dem Behälterinstallationsraum (73) montiert ist.

6. Kleidungsbehandlungsvorrichtung nach einem der Ansprüche 2 bis 5, wobei der Sensor (104) des Wasserabführungsstandsensors (101) auf einer Höhe angeordnet ist, die unteren Enden des Schwimmergehäuses (105) des Wasserabführungsstandsensors (101) entspricht, und der Schwimmer (102) des Wasserabführungsstandsensors (101) sich höher bewegt als der Sensor (104) des Wasserabführungsstandsensors (101), wenn der Stand des in dem Wasserversorgungsbehälter (80) gelagerten Wassers erhöht wird.

7. Kleidungsbehandlungsvorrichtung nach einem der Ansprüche 2 bis 6, wobei das Schwimmergehäuse (105) des Wasserabführungsstandsensors (101) auf einer Rückseite tiefer als das abgerundete obere Ende des Wasserabführungsbehälters (90) installiert ist.

8. Kleidungsbehandlungsvorrichtung nach einem der Ansprüche 2 bis 7, wobei der Sensor (104) des Wasserabführungsstandsensors (101) ferner bestimmt, ob der Wasserabführungsbehälter (90) in dem Behälterinstallationsraum (73) installiert ist.

9. Kleidungsbehandlungsvorrichtung nach einem der Ansprüche 2 bis 8, die ferner aufweist:
eine Steuereinheit (60), die in der Arbeitskammer (14) angeordnet ist und konfiguriert ist, um die Dampfeinheit (40) und die Wärmepumpeneinheit (50) basierend auf jedem Signal von dem Wasserversorgungsstandsensor (100) und dem Wasserabführungsstandsensor (101) zu steuern.

10. Kleidungsbehandlungsvorrichtung nach Anspruch 9, wobei die Kleidungsbehandlungsvorrichtung es unterlässt zu starten und die Steuereinheit (60) konfiguriert ist, um ein Wassermangelsignal auszugeben, wenn die Höhe des Schwimmers (102) des Wasserversorgungsstandsensors (100) niedriger als die Höhe des Sensors (104) des Wasserversorgungsstandsensors (100) vor dem Betrieb der Kleidungsbehandlungsvorrichtung ist, und
wobei die Steuereinheit (60) es während des Betriebs der Kleidungsbehandlungsvorrichtung bis zum Beenden unterlässt, das Wassermangelsignal auszugeben, obwohl die Höhe des Schwimmers (102) des Wasserversorgungsstandsensors (100) niedriger als die Höhe des Sensors (104) des Wasserversorgungsstandsensors (100) ist.

11. Kleidungsbehandlungsvorrichtung nach Anspruch 9 oder 10, wobei die Steuereinheit (60) konfiguriert ist, um zu bestimmen, dass das in dem Wasserversorgungsbehälter (80) gelagerte Wasser ausreicht, um die Kleidungsbehandlungsvorrichtung zu betreiben, wenn der Schwimmer (102) des Wasserversorgungsstandsensors (100) auf der entsprechenden Höhe des Sensors (104) des Wasserversorgungsstandsensors (100) angeordnet ist.

12. Kleidungsbehandlungsvorrichtung nach einem der Ansprüche 9 bis 11, wobei die Kleidungsbehandlungsvorrichtung es unterlässt zu starten und die Steuereinheit (60) konfiguriert ist, um ein Wassermangelsignal auszugeben, wenn die Höhe des Schwimmers (102) des Wasserabführungsstandsensors (101) höher als die Höhe des Sensors (104) des Wasserabführungsstandsensors (101) vor dem Betrieb der Kleidungsbehandlungsvorrichtung ist, und wobei die Steuereinheit (60) es während des Betriebs der Kleidungsbehandlungsvorrichtung bis zum Beenden unterlässt, das Wassermangelsignal auszugeben, obwohl die Höhe des Schwimmers (102) des Wasserabführungsstandsensors (101) höher als die Höhe des Sensors (104) des Wasserabführungsstandsensors (101) ist.

13. Kleidungsbehandlungsvorrichtung nach einem der Ansprüche 9 bis 12, wobei die Steuereinheit (60) konfiguriert ist, um zu bestimmen, ob das verbleibende Fassungsvermögen von Wasser in dem Wasserabführungsbehälter (90) ausreicht, um die Kleidungsbehandlungsvorrichtung zu betreiben, wenn der Schwimmer (102) des Wasserabführungsstandsensors (101) auf der entsprechenden Höhe des Sensors (104) des Wasserabführungsstandsensors (101) angeordnet ist.

14. Kleidungsbehandlungsvorrichtung nach einem der Ansprüche 1 bis 13, wobei der Behältermodulrahmen (71) vor einem Einlasskanal (34) installiert ist, der konfiguriert ist, um Luft von der Behandlungskammer (12) zu der Arbeitskammer (14) zu leiten, und konfiguriert ist, um einen in dem Behältermodulrahmen (71) definierten Behälterinstallationsraum (73) und die Arbeitskammer (14) zu unterteilen, und wobei der Wasserversorgungsbehälter (80) und der Wasserabführungsbehälter (90) getrennt in dem Behälterinstallationsraum (73) montiert sind.

15. Kleidungsbehandlungsvorrichtung nach Anspruch 14, wobei jede Vorderseite des Wasserversorgungsbehälters (80) und des Wasserabführungsbehälters (90) zu einem Benutzer freiliegt, wenn die Tür (20) offen ist, und jede Vorderseite des Wasserversorgungsbehälters (80) und des Wasserabführungsbehälters (90) der Innenseite der Tür (20) zugewandt ist, wenn die Tür (20) geschlossen ist.

## Revendications

1. Appareil de traitement de vêtements comprenant :
une armoire (10) divisée en une chambre de traitement (12) pour permettre d'y suspendre des vêtements, une chambre de cycle (14) pour permettre d'y installer une machinerie, et un châssis de module de cuve (71) configuré pour permettre d'y installer une cuve d'alimentation en eau (80) et une cuve de vidange (90) ;
une porte (20) pour ouvrir et fermer l'armoire (10) ;
une unité de vapeur (40) disposée dans la chambre de cycle (14) pour alimenter en vapeur la chambre de traitement (12) ;
une unité de pompe à chaleur (50) disposée dans la chambre de cycle (14) pour faire circuler de l'air dans la chambre de traitement (12) et conditionner l'air qui est mis en circulation ;
dans lequel la cuve d'alimentation en eau (80) est installée de façon séparable dans le châssis de module de cuve (71), et est raccordée à l'unité de vapeur (40), configurée pour alimenter en eau l'unité de vapeur (40), et
dans lequel la cuve de vidange (90) est installée de façon séparable dans le châssis de module de cuve (71), et est configurée pour stocker de l'eau condensée générée dans au moins l'une sélectionnée parmi la chambre de traitement (12) et l'unité de pompe à chaleur (50) ; **caractérisé en ce qu'**il comprend en outre
un capteur de niveau d'alimentation en eau (100) configuré pour détecter un niveau d'eau stockée dans la cuve d'alimentation en eau (80) ;
un capteur de niveau de vidange (101) configuré pour détecter un niveau d'eau stockée dans la cuve de vidange (90),
et le capteur de niveau de vidange (101) est situé plus haut que le capteur de niveau d'alimentation en eau (100).

2. Appareil de traitement de vêtements selon la revendication 1, dans lequel chacun du capteur de niveau d'alimentation en eau (100) et du capteur de niveau de vidange (101) comprend :
un caisson à flotteur (105) fixé dans chacune de la cuve d'alimentation en eau (80) et de la cuve de vidange (90) ;
un flotteur (102) installé dans le caisson à flotteur (105) de sorte que le flotteur (102) soit mobile vers le haut et vers le bas dans le caisson à flotteur (105) par flottaison ; et
un capteur (104) installé dans le châssis de module de cuve (71) et configuré pour détecter une force magnétique du flotteur (102).

3. Appareil de traitement de vêtements selon la revendication 2, dans lequel le capteur (104) du capteur de niveau d'alimentation en eau (100) est situé à une hauteur correspondante vers l'extrémité supérieure du caisson à flotteur (105) du capteur de niveau d'alimentation en eau (100), et le flotteur (102) du capteur de niveau d'alimentation en eau (100) se déplace plus bas que le capteur (104) du capteur de niveau d'alimentation en eau (100) lorsque le niveau d'eau stockée dans la cuve d'alimentation en eau (80) est abaissé.

4. Appareil de traitement de vêtements selon la revendication 2 ou 3, dans lequel le caisson à flotteur (105) de la cuve d'alimentation en eau (80) est installé au niveau d'une surface inférieure vers l'intérieur d'un côté arrière de la cuve d'alimentation en eau (80).

5. Appareil de traitement de vêtements selon l'une quelconque des revendications 2 à 4, dans lequel le capteur (104) du capteur de niveau d'alimentation en eau (100) détermine en outre si la cuve d'alimentation en eau (80) est montée dans l'espace d'installation de cuve (73).

6. Appareil de traitement de vêtements selon l'une quelconque des revendications 2 à 5, dans lequel le capteur (104) du capteur de niveau de vidange (101) est situé à une hauteur correspondante vers des extrémités inférieures du caisson à flotteur (105) du capteur de niveau de vidange (101), et le flotteur (102) du capteur de niveau de vidange (101) se déplace plus haut que le capteur (104) du capteur de niveau de vidange (101) lorsque le niveau d'eau stockée dans la cuve d'alimentation en eau (80) est augmenté.

7. Appareil de traitement de vêtements selon l'une quelconque des revendications 2 à 6, dans lequel le caisson à flotteur (105) du capteur de niveau de vidange (101) est installé au niveau d'un côté arrière plus bas que l'extrémité supérieure arrondie de la cuve de vidange (90).

8. Appareil de traitement de vêtements selon l'une quelconque des revendications 2 à 7, dans lequel le capteur (104) du capteur de niveau de vidange (101) détermine en outre si la cuve de vidange (90) est montée dans l'espace d'installation de cuve (73).

9. Appareil de traitement de vêtements selon l'une quelconque des revendications 2 à 8, comprenant en outre :
une unité de commande (60) située dans la chambre de cycle (14) et configurée pour commander l'unité de vapeur (40) et l'unité de pompe à chaleur (50), d'après chaque signal provenant de chacun du capteur de niveau d'alimentation en eau (100) et du capteur de niveau de vidange (101).

10. Appareil de traitement de vêtements selon la revendication 9, dans lequel l'appareil de traitement de vêtements ne parvient pas à démarrer et l'unité de commande (60) est configurée pour fournir en sortie un signal de déficit d'eau lorsque la hauteur du flotteur (102) du capteur de niveau d'alimentation en eau (100) est plus basse qu'une hauteur du capteur (104) du capteur de niveau d'alimentation en eau (100) avant le fonctionnement de l'appareil de traitement de vêtements, et dans lequel l'unité de commande (60) ne parvient pas à fournir en sortie un signal de déficit d'eau avant la fin, même si la hauteur du flotteur (102) du capteur de niveau d'alimentation en eau (100) est plus basse que la hauteur du capteur (104) du capteur de niveau d'alimentation en eau (100) pendant le fonctionnement de l'appareil de traitement de vêtements.

11. Appareil de traitement de vêtements selon la revendication 9 ou 10, dans lequel l'unité de commande (60) est configurée pour déterminer que l'eau stockée dans la cuve d'alimentation en eau (80) est suffisante pour faire fonctionner de l'appareil de traitement de vêtements lorsque le flotteur (102) du capteur de niveau d'alimentation en eau (100) est situé à la hauteur correspondante du capteur (104) du capteur de niveau d'alimentation en eau (100).

12. Appareil de traitement de vêtements selon l'une quelconque des revendications 9 à 11, dans lequel l'appareil de traitement de vêtements ne parvient pas à démarrer et l'unité de commande (60) est configurée pour fournir en sortie un signal de déficit d'eau lorsque la hauteur du flotteur (102) du capteur de niveau de vidange (101) est plus haute que la hauteur du capteur (104) du capteur de niveau de vidange (101) avant le fonctionnement de l'appareil de traitement de vêtements, et dans lequel l'unité de commande (60) ne parvient pas à fournir en sortie un signal de déficit d'eau avant la fin, même si la hauteur du flotteur (102) du capteur de niveau de vidange (101) est plus haute que la hauteur du capteur (104) du capteur de niveau de vidange (101) pendant le fonctionnement de l'appareil de traitement de vêtements.

13. Appareil de traitement de vêtements selon l'une quelconque des revendications 9 à 12, dans lequel l'unité de commande (60) est configurée pour déterminer si la capacité en eau restante dans la cuve de vidange (90) est suffisante pour faire fonctionner l'appareil de traitement de vêtements lorsque le flotteur (102) du capteur de niveau de vidange (101) est situé à la hauteur correspondante du capteur (104) du capteur de niveau de vidange (101).

14. Appareil de traitement de vêtements selon l'une quelconque des revendications 1 à 13, dans lequel le châssis de module de cuve (71) est installé devant un conduit d'entrée (34) configuré pour guider de l'air de la chambre de traitement (12) à la chambre de cycle (14), et est configuré pour diviser un espace d'installation de cuve (73) défini dans le châssis de module de cuve (71) et la chambre de cycle (14), et dans lequel chacune de la cuve d'alimentation en eau (80) et de la cuve de vidange (90) est montée de façon séparable dans l'espace d'installation de cuve (73).

15. Appareil de traitement de vêtements selon la revendication 14, dans lequel chaque côté avant de la cuve d'alimentation en eau (80) et de la cuve de vidange (90) est exposé à un utilisateur lorsque la porte (20) est ouverte, et chaque côté avant de la cuve d'alimentation en eau (80) et de la cuve de vidange (90) est face au côté interne de la porte (20) lorsque la porte (20) est fermée.
